# EUROPEAN PATENT SPECIFICATION

(11) **EP 1 196 167 B1**
(45) Date of publication and mention of the grant of the patent: **19.04.2006**
(21) Application number: 00948576.4
(22) Date of filing: 30.06.2000
(51) Int. Cl.: A61K 31/40, A61K 31/4439, A61K 31/444, A61K 31/506, A61K 31/415, A61K 31/4545, A61K 31/00

(54) **Use of p38 MAPK inhibitors in the treatment of ophthalmic conditions**
Verwendung von p38 MAPK Inhibitoren in der Behandlung von Augenkrankheiten
Utilisation des inhibiteurs p38 MAPK pour des maladies ophthalmiques

(30) Priority: 02.07.1999 US 142341 P
(43) Date of publication of application: 17.04.2002
(73) Proprietor: Lipton, Stuart A., Rancho Santa Fe, CA 92067-4018 (US)
(72) Inventor: Lipton, Stuart A., Rancho Santa Fe, CA 92067-4018 (US)
(74) Representative: Hitchcock, Esmond Antony
(86) International application number: PCT/US2000/018385
(87) International publication number: WO 2001/001986

(56) References cited:
- WO-A-98/27098
- WO-A-99/00357
- WO-A-99/61039
- US-B1- 6 288 089
- NAGARKATTI D S ET AL: "ROLE OF P38 MAP KINASE IN MYOCARDIAL STRESS" JOURNAL OF MOLECULAR AND CELLULAR CARDIOLOGY, ACADEMIC PRESS, LONDON, GB, vol. 30, no. 8, August 1998 (1998-08), pages 1651-1664, XP000857687 ISSN: 0022-2828
- MA X L ET AL: "INHIBITION OF P38 MITOGEN-ACTIVATED PROTEIN KINASE DECREASES CARDIOMYOCYTE APOPTOSIS AND IMPROVES CARDIAC FUNCTION AFTER MYOCARDIAL ISCHEMIA AND REPERFUSION" CIRCULATION, AMERICAN HEART ASSOCIATION, DALLAS, TX, US, vol. 99, no. 13, 6 April 1999 (1999-04-06), pages 1685-1691, XP000857740 ISSN: 0009-7322
- LIEU ET AL: "Role of mitogen-activated protein kinase in Taxol-induced apoptosis in human leukemic U937 cells" CELL GROWTH AND DIFFERENTIATION, THE ASSOCIATION, PHILADELPHIA, PA, US, vol. 9, no. 9, September 1998 (1998-09), pages 767-776, XP000909267 ISSN: 1044-9523
- DATABASE CA ON STN, CHEMICAL ABSTRACTS SERVICE (COLUMBUS, OHIO, USA) ACCESSION NO. 129:14783 COURTNEY ET AL.: 'p38 Mitogen-activated protein kinase-dependent and independent intracellular signal transduction pathways leading to apoptosis in human neutrophils', XP002932013 & J. BIOL. CHEM., vol. 273, no. 14, June 1998, pages 8389 - 8397

## Description

This invention relates to the treatment of nervous system disorders, including those mediated by the NMDA receptor, e.g., responsive to glutamate.

HIV-1 glycoprotein gp120, the coat protein of HIV, has been shown to produce neuronal damage and apoptosis in both rodent and human cell cultures. See, e.g., Brenneman et al., *Nature* 335:639-642 (1988); Dreyer et al., *Science* 248:364-367 (1990); and Müller et al., *Eur. J. Pharmacol.* 226:209-214 (1992). Transgenic mice expressing gp120 developed neuropathological features resembling in many ways HIV-mediated dementia. See Toggas et al., *Nature* 367:188-193 (1994). Although FHV-1 itself does not apparently infect neurons, HIV-1 infection can occur in other cells in the brain (e.g., macrophages/microglia) and this can lead to neuronal injury, damage, or death by apoptosis in the brains of patients with AIDS. In addition to neurons in the brain, HIV-1 infection also affects other types of nerve cells, e.g., retinal ganglion cells and can lead to loss of vision.

p38 mitogen-activated protein kinase (MAPK) inhibitors are well known and have been proposed for treatment of a number of different specific disorders. See: Nagarkatti, D.S., *et al.* "Role of p38 MAP Kinase in Myocardial Stress", J Mol Cell Cardiol 30, 1651-1664 (1998); Ma, X.L., *et al.* "Inhibition of p38 Mitogen-Activated Protein Kinase Decreases Cardiomyocyte Apoptosis and Improves Cardiac Function After Myocardial Ischemia and Reperfusion", American Heart Association Vol. 99, No. 13, 1999, 1685-1691; Lieu, C-H, *et al.* "Role of Mitogen-activated Protein Kinase in Taxol-Induced Apoptosis in Human Leukemic U937 Cells", Cell Growth & Differentiation Vol. 9, 1998, 767-776; WO 99/00357 Vertex Pharmaceuticals Inc.; WO 98/27098 Vertex Pharmaceuticals Inc.; WO99/61039 Max-Planck-Gesellschaft zur Förderung der Wissenschaften E.V.; US 6288089 Zawada *et al.;* and Courtney *et al.* "CA on STN2, Chemical Abstracts Service, Accession No. 129:14783.

The present invention proposes the use of p38 MAPK inhibitors in medicaments for treating a number of specific medical conditions for which such inhibitors have not previously been employed, namely for glaucoma, optic neuropathy, optic neuritis, retinal ischemia, laser induced optic damage, or surgery-or trauma-induced proliferative vitreoretinopathy, neuronal injury or apoptosis involving retinal ganglion cells, or a medical condition which causes nitrosative or oxidative stress to retinal ganglion cells by excessive activation of excitatory amino acid receptors or the generation of free radicals.

According to a first aspect thereof, the present invention proposes use of a p38 mitogen-activated protein kinase (MAPK) inhibitor in the manufacture of a medicament for treating a patient suffering from glaucoma, optic neuropathy, optic neuritis, retinal ischemia, laser induced optic damage, or surgery- or trauma-induced proliferative vitreoretinopathy; a patient suffering neuronal injury or apoptosis involving retinal ganglion cells; or a patient suffering from a medical condition which causes nitrosative or oxidative stress to retinal ganglion cells by excessive activation of excitatory amino acid receptors or the generation of free radicals.

Suitably, the p38 MAPK inhibitor is a compound with the ability to inhibit TNF- a in known models, such as the model using human peripheral blood nomonuclear cells that are stimulated by LPS as described in Henry et al., Bioorg. and Med. Chem. Lett. 8:3335-3340 (1998), hereby incorporated by reference. Such inhibition may be demonstrated by an IC₅₀ of no more than 500 nM in such assays. Strong inhibit ion is most preferably characterized by an IC₅₀ of no more than 100nM when evaluated in such assays.

In one embodiment, the p38 MAPK inhibitor is of formula (I): 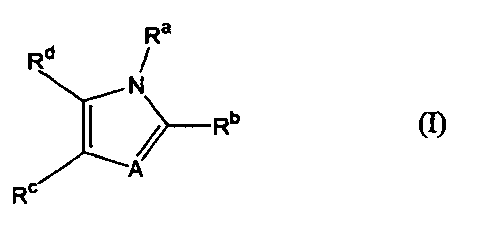 A is N or CR^{α}. Each of R^{α}, R^{a}, R^{b}, and R^{c}, independently, is hydrogen, alkyl, hydroxyl, alkoxy, aryloxy, heteroaryloxy, thio, amino, amide, carboxyl, ester, amide, halo, cycloalkyl, heterocycloalkyl, aryl, heteroaryl, aralkyl, or heteroaralkyl, each of cycloalkyl, heterocycloalkyl, aryl, and heteroaryl being optionally substituted with alkyl, hydroxyl, alkoxy, aryloxy, heteroaryloxy, thio, amino, amide, carboxyl, ester, alkylsulfinyl, or halo. R^{c} and R^{d} optionally join together to form cycloalkyl, heterocycloalkyl, aryl, or heteroary; each of which being optionally substituted with alkyl, hydroxyl, alkoxy, aryloxy, heteroaryloxy, thio, amino, amide, carboxyl, ester, alkylsulfinyl, or halo.

Use of a salt of the p38 MAPK inhibitor of formula (I) is also within the scope of this invention. For example, a salt can form between an positively charged amino substituent (e.g., -N⁺(CH₃)₃) with a negatively charged counterion (e.g., chloride, bromide, nitrate, or sulfate). As another example, a negatively charged carboxylate substituent can form a salt with a positively charged counterion such as sodium ion, potassium ion, or a magnesium ion.

In one embodiment, the p38 MAPK inhibitor is an imidazole (i.e., A is N) with R^{a} being hydrogen, cycloalkyl, heterocycloalkyl, aryl, heteroaryl, aralkyl, or heteroaralkyl (e.g., piperidine); R^{b} being hydrogen, aryl, or heteroaryl (e.g., 4-alkylsulfinylphenyl); R^{c} being hydrogen, aryl, or heteroaryl (e.g., 4-halophenyl); and R^{d} is 4-pyridyl.

In another embodiment, the p38 MAPK inhibitor is an pyrrole (i.e., A is CR^{α} wherein R^{a} can be hydrogen, aryl, or heteroaryl). R^{c} and R^{d} can join together to form heteroaryl (e.g., pyridine) which is optionally substituted with alkyl, alkoxy, aryloxy, amino, or halo (e.g., -OC₃ or NH₂).

The p38 MAPK inhibitor can be selected from one or more of the following: SmithKline Beecham Pharmaceuticals (King of Prussia, PA) triarylimidazole or triarylpyrrole compounds such as Smith Kline drug no. SB 203580 (i.e., 4-(4-fluorophenyl)-2-(4-methylsulfinylphenyl)-5-(4-pyridyl)-*1H*-imidazole), SB 202190 (i.e., 4-(4-fluorophenyl)-2-(4-hydroxylphenyl)-5-(4-pyridyl)-*1H*-imidazole), SB 220025 (i.e., 5-(2-amino-4-pyrimidinyl)-4-(4-fluorophenyl)-1-(4-piperidinyl)-*1H-*imidazole), SmithKline Beecham drug no. SB 239063, SC 68376 (i.e., 2-methyl-4-phenyl-5-(4-pyridyl)oxazole, available from Alexis Biochemicals, San Diego, CA), SmithKline French drug no. SKF-104,351, SKF-86002 (i.e., 6-(4-fluorophenyl)-2,3-dihydro-5-(4-pyridyl)imidazo-[2,1-b]-thiazole); R.W. Johnson Pharmaceutical Research Institute (Raritan, NJ) pyrrolopyridine drugs such as RWJ 68354 (i.e., 6-amino-2-(4-fluorophenyl)-4-methoxy-3-(4-pyridyl)-*1H-*pyrrolo[2,3-b]pyridine); Vertex Pharmaceuticals drug no. VK-19911 (i.e., 1-(4-piperidinyl)-4-(4-fluorophenyl)-5-(4-pyridyl)imidazole); or derivatives or congeners of each of the just-mentioned compounds.

The patient being treated is suffering from glaucoma or other optic neuropathies such as optic neuritis, retinal ischemia, laser induced optic damage, proliferative vitreoretinopathy that is induced, e.g. by surgery or trauma; from neuronal injury or apoptosis involving retinal ganglion cells; or from a medical condition which causes nitrosative or oxidative stress to retinal ganglion cells by excessive activation of excitatory amino acid receptors or the generation of free radicals.

The neuronal apoptosis may be induced by HIV infection (e.g., by gp120 protein), an α-chemokine (e.g., SDF-1), or a combination of both.

In a particular case, the invention is concerned with treating glaucoma. A patient suffering from glaucoma may be administered with an effective amount of a p38 mitogen-activated protein kinase (MAPK) inhibitor. In one embodiment, the p38 MAPK inhibitor is 4-(4-fluorophenyl)-2-(4-methylsulfinylphenyl)-5-(4-pyridyl)-*1H*-imidazole, 4-(4-fluorophenyl)-(4-hydroxylphenyl)-5-(4-pyridyl)-*1H-*imidazole, 5-(2-amino-4-pyrimidinyl)-4-(4-fluorophenyl)-1-(4-piperidinyl)-*1H-*imidazole, 2-methyl-4-phenyl-5(-4-pyridyl)oxazole, SmithKline French drug no. SKF-104,351,
6-(4-fluorophenyl)-2,3-dihydro-5-(4-pyridyl)imidazo-[2,1-b]-thiazole, 6-amino-2-(4-fluorophenyl)-4-methoxy-3-(4-pyridyl)-*1H*-pyrrolo[2,3-b]pyridine, or 1-(4-piperidinyl)-4-(4-fluorophenyl)-5-(4-pyridyl)-*1H*-imidazole.

As used herein, alkyl is a straight or branched hydrocarbon chain containing 1 to 8 carbon atoms. Examples of alkyl include, but are not limited to, methyl, ethyl, propyl, isopropyl, butyl, isobutyl, sec-butyl, tert-butyl, n-pentyl, and 2-methylhexyl.

By cycloalkyl is meant a cyclic alkyl group containing 3 to 8 carbon atoms. Some examples of cycloalkyl are cyclopropyl, cyclopentyl, cyclohexyl, cycloheptyl, adamantyl, and norborayl. Heterocycloalkyl is a cycloalkyl group containing 1-3 heteroatoms such as nitrogen, oxygen, or sulfur. Examples of heterocycloalkyl include piperidinyl, piperazinyl, tetrahydropyranyl, tetrahydrofuryl, and morpholinyl.

As used herein, aryl is an aromatic group containing 6-12 ring atoms and can contain fused rings, which may be saturated, unsaturated, or aromatic. Examples of an aryl group include phenyl, naphthyl, biphenyl, phenanthryl, and anthracyl. Heteroaryl is aryl containing 1-3 heteroatoms such as nitrogen, oxygen, or sulfur and can contain fused rings. Some examples of heteroaryl are pyridyl, fiuanyl, pyrrolyl, thienyl, thiazolyl, oxazolyl, imidazolyl, indolyl, benzofuranyl, and benzthiazolyl.

Note that an amino group can be unsubstituted, mono-substituted, or disubstituted. It can be substituted with groups such as alkyl, cycloalkyl, heterocycloalkyl, aryl, heteroaryl, aralkyl, or heteroaralkyl. Halo refers to fluoro, chloro, bromo, or iodo.

Other features and advantages will be apparent from the following Detailed Description.

In the drawings:
FIG. 1(A) is a bar graph of neuronal apoptosis in the presence of gp120, the β-chemokine RANTES, or a combination of gp120 and RANTES.
FIG. 1(B) is a bar graph of neuronal apoptosis in the presence of gp120, the β-chemokine MIP-1β, or a combination of gp120 and MIP-1β.
FIG. 2 is a bar graph of neuronal apoptosis in the presence of gp120, the α-chemokines SDF-1β (20 nm), SDF-1β (50 nm), SDF-1α (50 nm), and a combination of gp120 and each of SDF-1β (20 nm), SDF-1β (50 nm), and SDF-1α (50 nm).
FIG. 3(A) is a bar graph of neuronal apoptosis in the presence of gp120, the tri-peptide TKP, or a combination of gp120 and TKP.
FIG. 3(B) is a bar graph of neuronal apoptosis in the presence of gp120, SDF-1β, or a combination of gp120 and SDF-1β.
FIG. 3(C) is a bar graph of nitrite (reflective of nitric oxide) production in the presence of cytokines or a combination of TKP and cytokines.
FIG. 4(A) is a bar graph of neuronal apoptosis in the presence of gp120, Smith Kline drug no. SB 203580, or a combination of gp120 and SB 203580.
FIG. 4(B) is a bar graph of neuronal apoptosis in the presence of SDF-1β, Smith Kline drug no. SB 203580, or a combination of SDF-1β and SB 203580.
FIG. 5 is a graph showing the effects of axotomy on the density of RGCs.
FIG. 6 is a bar graph showing neuroprotective effect of SB 203580.
FIG. 7 is a bar graph showing inhibition of p38 activity protects cultured RGCs from *N*-methyl-D-aspartate (NMDA)-induced apoptosis.
FIG. 8 is a bar graph showing neuroprotective effect of MK801 against axotomy-induced RGC apoptosis *in vivo.*

As the detailed description below explains, p38 mitogen-activated protein kinase (MAPK) inhibitors can be shown to reduce neuronal injury, damage, or death, e.g., neuronal apoptosis induced by HIV-infection. Based on this work, it has also ben shown that such inhibitors provide an effective treatment for glaucoma and a number of related retinal neurodegenerative diseases.

Without intending to be bound by any theory, a p38 mitogen-activated protein kinase (MAPK) inhibitor protects neurons from damage or death from apoptosis resulting from HIV infection based on the following findings. All publications recited herein are hereby incorporated by reference in their entirety.

### Rat Cerebrocortical Cultures as

### In Vitro Model for Neuronal Apoptosis Assays

Although some gp120 proteins from HIV-1 can signal directly via chemokine receptors on neuronal cell lines and on isolated rodent neurons, the importance of cell-cell interactions in the brain mandates that disease pathogenesis *in vitro* be approached in a culture system that recapitulates the type and proportion of cells normally found in brain such as neurons, astrocytes, and macrophages/microglia.

The model used in the assays described below, i.e., rat cerebrocortical cultures, was prepared from embryos of Sprague-Dawley rats at day 15 to 17 of gestation. See Lei et al., *Neuron* 8:1087-1099 (1992) and Lipton et al., *Nature* 364:626-632 (1993). Cultures were used for experiments after 17 to 24 days in culture. These cultures contain neurons, astrocytes, and macrophages/microglia, as determined with specific immunolabeling. Prior to some experiments, macrophages/microglia or neurons were depleted from the cultures by exposure to 7.5 mM L-leucine methyl ester or 2 mM N-methyl-D-aspartate (NMDA), respectively. See Lipton, *NeuroReport* 3:913-915 (1992). Absence of macrophages/microglia or neurons in these cultures was confirmed immunocytochemically, using antibodies to ED-1 and microtubule-associated protein-2 (MAP-2), respectively. In some experiments, the Griess reaction was used to measure nitrite levels in the culture medium as an index of nitric oxide (NO) release. See Chao et al., *Glia* 16:276-284 (1996).

### Neuronal Apoptosis Assays

Cultures were transferred into Earle's Balanced Salt Solution (BBSS) and incubated for 24 hours with gp120, chemokines, Tuftsin fragment 1-3, i.e., Thr-Lys-Pro (TKP), p38 MAPK inhibitor SB 203580 (i.e., 4-(4-fluorophenyl)-2-(4-methylsulfinylphenyl)-5-(4-pyridyl)imidazole, Calbiochem, San Diego, CA), or combinations thereof. Chemokines or TKP were applied for 5 minutes and SB 203580 for 15 minutes prior to gp120 exposure.

TKP was obtained from Sigma (St. Louis, MO). Recombinant human MIP-1β. SDF-1α, SDF-1β, and recombinant rat RANTES were purchased from R&D systems (Minneapolis, WI) and Endogen (Wobum, MA), respectively. HIV-1 envelope glycoprotein gp120 from the strain SF2 was obtained from the NIH AIDS Research and Reference Reagent Program, Division of AIDS, NIAID, NIH. See Scandella et al., AIDS Res. Hum. Retroviruses 9:1233-1244 (1993). Additional gp120s from HTV-1 strains IIIB and RF2 were obtained from Genentech and the National Cancer Institute, respectively. TNFα, IFNγ, and IL-1β were from Genzyme (Cambridge, MA), Gibco BRL (Grand Island, NY) and Endogen (Woburn, MA), respectively.

### Assessment of Neuronal Apoptosis

Neuronal apoptosis in the above-described assays was assessed using a variety of techniques, e.g., staining of permeabilized cells with propidium iodide to determine apoptotic morphology, and a neuron-specific antibody (against NeuN or MAP-2) to identify cell type. In brief, cells were fixed for 5 minutes with ice-cold acetone at -20°C and, after three washes in PBS, for 4 minutes with 2% (w/v) paraformaldehyde solution in PBS at room temperature (RT). Acetone-paraformaldehyde-fixed cells were permeabilized using 0.2% Tween 20 in PBS (PBST), and non-specific binding sites were blocked by incubation for 1 hour with a 10% solution of heat-inactivated goat serum in PBST. To specifically stain neurons, cells were then incubated for 4 hours at RT or overnight at 4°C with 1:500 dilutions of anti-MAP-2 (Sigma) or anti-NeuN monoclonal antibody (mAb; Chemicon, Temecula, CA). Their respective non-specific isotype antibodies served as controls. After three washes, the cells were incubated in a secondary polyclonal antibody (pAb) conjugated either to fluorescein isothiocyanate (FITC) or to horseradish peroxidase (HRP). In the case of HRP-coupled pAb, diaminobenzidine (DAB) served as the color substrate developed by incubation in a mixture of 1 mg/ml DAB and 0.8% H₂O₂ at a ratio of 3:1. Cellular nuclei were subsequently stained with 20 mg/ml propidium iodide for 5 minutes in the dark, and then coverslips were mounted on glass slides. Experiments were replicated at least 3 times with triplicate values in each experiment. Statistical significance was judged by an analysis of variance (ANOVA) followed by a Scheffé or Bonferroni/Dunn post hoc test.

### β-chemokines abrogate gp120-induced

### neuronal apoptosis; α-chemokines induce neuronal apoptosis

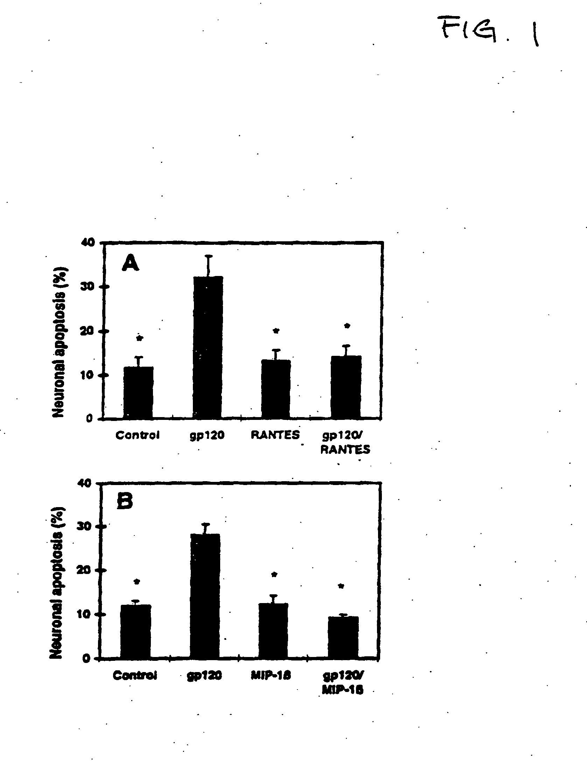

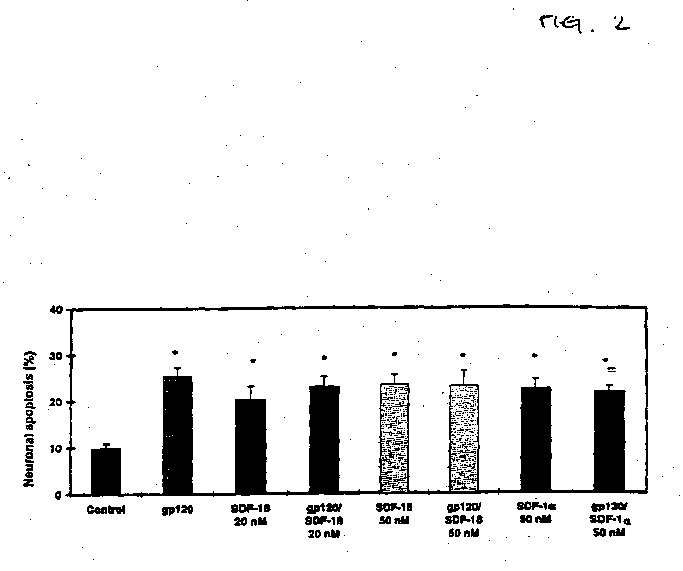

Referring to FIG. 1(A) and FIG. 1(B), neuronal apoptosis induced by gp120 (200 pM) was abrogated by the presence of β-chemokines, i.e., RANTES and MIP-1β (each at 20 nM), whilst BSA (0.001% =144 nM) and the presence of α-chemokines, i.e., SDF-1α or SDF-1β (20-50 nM), did not reduce neuronal damage or apoptosis. In fact, these α-chemokines increased neuronal apoptosis (approximately 2-fold increase in neuronal apoptosis compared to control). See Fig. 2. MIP-1β and RANTES inhibit the neurotoxic effect of gp120SF2 in an indirect manner since RANTES binds to the β-chemokine receptors CCR1, CCR3, and CCR5, and MIP-1β binds CCR5 (or a functional rat homologue), whereas gp120SF2 (andSDF-1α/β) interact with the α-chemokine receptor CXCR4. Note that although gp120SF2 may also interact to a lesser degree with the β-chemokine receptor CCR5 on some transfected cell lines, this has not been shown to occur on primary cells, as used here.

It should be pointed out that rodent cerebrocortical cultures are a suitable model system to study these actions of gp 120 since these species express CXCR4 homologues which, like the human CXCR4, are capable of mediating HIV-1 infection via gp120 binding.

### Mode of Action of gp120 and chemokines

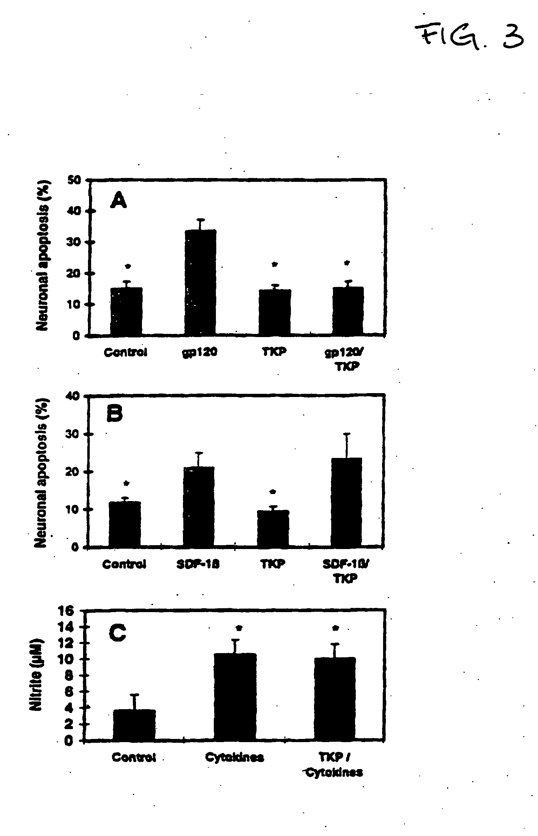

To investigate whether the neuroprotective effect of these β-chemokines and the neurotoxic effect of gp120 are mediated by macrophages, astrocytes, neurons, or by simultaneous action on two or all three cell types, the macrophage inhibitory tripeptide Thr-Lys-Pro (TKP) was used in the assay. TKP has been shown to specifically prevent activation of macrophages/microglia and subsequent release of their toxic factors both *in vitro* and *in vivo,* whereas control peptides have no effect. See, e.g., Auriault et al., *FEBS Lett.* 153:11-15 (1983). In our assays, TKP (50 µM) protected neurons from gp120-induced apoptosis. See FIG. 3(A). In contrast, SDF-1β-induced neuronal apoptosis was not abrogated by TKP. See FIG. 3(B).

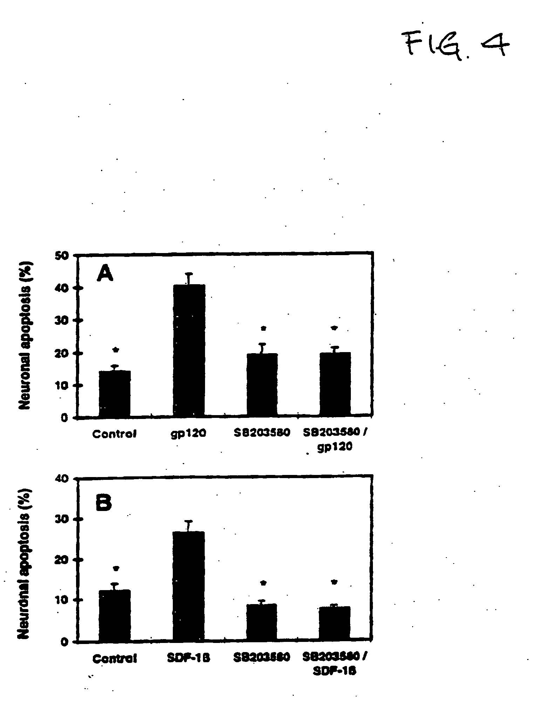

Several lines of evidence confirmed prior reports that TKP exerted its inhibitory effect specifically on macrophages/microglia, and not on astrocytes or neurons. For example, in the absence of macrophages/microglia, TKP did not inhibit NO release by cytokine-activated astrocytes. See FIG. 4(C), and TKP did not interfere with NMDA-induced neuronal apoptosis. These findings indicate that activated macrophages are necessary for gp 120-induced, but not α-chemokine-induced neuronal apoptosis.

The number of HIV-1 infected cells in the brain is relatively small, and productively infected cells are exclusively of monocytoid lineage. See Lipton and Gendelman, *N. Engl. J. Med.* 332:934-940 (1995). This suggests that HIV-1 initiates a neurodegenerative process that entails amplification to produce pronounced CNS injury. Indeed, in culture systems of both rodent and human brain, HTV-1 infected or gp120-stimulated macrophages and microglia have been found to release neurotoxins that contribute to the neurodegenerative process, at least in part, by excessive stimulation of the NMDA subtype of glutamate receptor. The fact that gp120-transgenic mice manifest neuronal damage resembling that found both in rodent cultures and in human brain with HIV-associated dementia indicates that, even in the absence of intact HIV-1, a fragment of the virus is sufficient to trigger important aspects of this amplification cascade in the neurodegenerative process in our *in vitro* system, which therefore has relevance to *in vivo* pathogenicity.

### Inhibition of p38 mitogen-activated protein kinase

### ameliorates both gp120- and SDF-1-induced neuronal apoptosis

In another series of experiments, we assayed a variety of inhibitors of intracellular signaling cascades for their ability to prevent neuronal apoptosis associated with gp120. These inhibitors included Parke-Davis drug no. PD 98059 (2 µM) (i.e., 2'-amino-3'-methoxyflavone; Calbiochem, La Jolla, CA) which inhibits extracellular regulated kinase (ERK) MAPK, pyrrolidine dithiocarbamate (PDTC, 5 µM) which inhibits NF-kB, and SB 203580 (10 µM) which specifically inhibits p38 MAPK. Among these compounds, only SB 203580 substantially attenuated gp120SF2-induced neuronal apoptosis. See FIG. 4(A). This supports the conclusion that the p38 MAPK pathway is involved in the gp120-activated death signaling. Inhibition of p38 MAPK also ameliorated SDF-1 neurotoxicity. See FIG. 4(B). This indicates that the neurotoxic processes initiated by both gp120SF2 and SDF-1 use the common MAPK signaling pathway which involves p38 MAPK. Since SDF-1-induced neurotoxicity occurs in the virtual absence of macrophages/microglia, p38 MAPK must be activated as a stress response in neurons or astrocytes. However, we cannot exclude the possibility that gp120 and SDF-1 also activate p38 in macrophages/microglia. In fact, this is likely to occur since immunocytochemical experiments in our culture system have revealed activated (diphosphorylated) p38 in both neurons and macrophages after gp120 stimulation.

### Other p38 MAPK inhibitors

There are other p38 MAPK inhibitors which inhibit the signaling pathway in a similar manner as SB 203580. These inhibitors include SmithKline Beecham Pharmaceuticals (King of Prussia, PA) triarylimidazole or triarylpyrrole compounds such as Smith Kline drug no. SB 202190 (i.e., 4-(4-fluorophenyl)-2-(4-hydroxylphenyl)-5-(4-pyridyl)imidazole), SB 220025 (i.e., 5-(2-amino-4-pyrimidinyl)-4-(4-fluorophenyl)-1-(4-pipendinyl)imidazole), SmithKline Beecham drug no. SB 239063, SmithKline French drug no. SKF-104,351; R.W. Johnson Pharmaceutical Research Institute (Raritan, NJ) pyrrolopyridine drugs such as RWJ 68354 (i.e., 6-amino-2-(4-fluorophenyl)-4-methoxy-3-(4-pyridyl)-1H-pyrrolo[2,3-b]pyridine); Vertex Pharmaceuticals drug no. VK-19911 (i.e., 1-(4-piperidinyl)-4-(4-fluorophenyl)-5-(4-pyridyl)imidazole); Alexis Biochemicals drug no. SC 68376 (i.e., 2-methyl-4-phenyl-5-(4-pyridyl)oxazole); and derivatives and congeners of each of the just-mentioned compounds.

These p38 MAPK inhibitors can be prepared by a number of different methods. For example, these inhibitors can be prepared by Fisher indole synthesis as described in Henry et al., *Bioorganic & Medicinal Chemistry Letters* 8:3335-40 (1998). See also Gallagher et al., *Bioorganic & Medicinal Chemistry Letters* 5(1):49-64 (1997) and Henry et al., *J. Med. Chem.* 22:4196-8 (1998).

### Assay for Treating Glaucoma

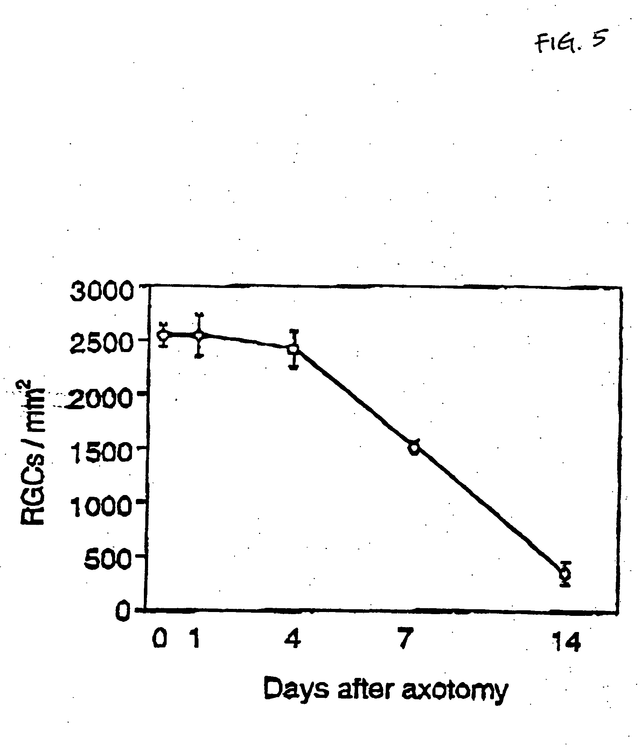

As a model for studying treatment of glaucoma, damaged (axotomized) retinal ganglion cells were employed. Specifically, transection of the optic nerve close to the cell body causes apoptotic death of retinal ganglion cells (RGCs), similar to that found in glaucoma. In adult rats, RGCs were retrogradely labeled with Fluoro-Gold™ (i.e., hydroxystilbamidine) injected into the superior colliculi. Three days later, the left optic nerve was intraorbitally transected 1 mm from the globe. One day after axotomy, no remarkable change in the fluorescence pattern of RGCs was seen compared to that of controls. RGC density, as assessed by the number of fluorescently-labeled cells, began to decline between 4 and 7 days post axotomy. By 14 days, a substantial number of fluorescently-labeled RGCs appeared to have been lost. Morphologically, in whole-mounted retinas stained with cresyl violet, ganglion cell layer (GCL) of unlesioned control retinas manifest no apoptotic profiles or pyknotic nuclei. In contrast, by 10 days after axotomy, multiple degenerating cell bodies with pyknotic nuclei were apparent in the GCL, and the cell density in this layer appeared to be greatly diminished. When hydroxystilbamidine fluorescence in the GCL was quantified, in unlesioned control retinas the mean RGC density was 2533 ± 104 cells/mm² (mean ± SD). Within 14 days of axotomy, the mean RGC density decreased to 348 ± 107 (14% of control). See Fig. 5.

One day after axotomy, activated (di-phosphorylated) p38 was visualized by immunocytochemistry in the RGC layer, but not in control retinas. By Western blotting, phosphorylated p38 was first detected 12 hours after axotomy and reached a maximum at 24 hours before decreasing. SB 203580 was administered intravitreally at the time of axotomy, and repeated at 5 days and 10 days.

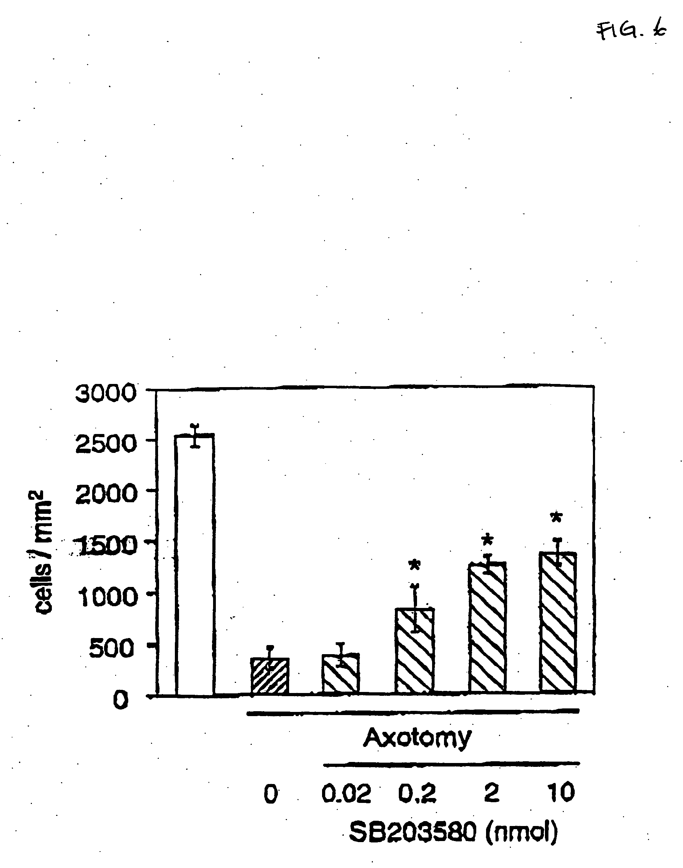

Assayed 14 days post axotomy, SB203580 increased the number of surviving RGCs in a dose-dependent manner. See Fig. 6. A significant degree of neuroprotection was observed after injection of as little as 0.2 nmol of SB203580, corresponding to an intravitreal concentration of 1.6 µM, given the volume of the rat vitreous has been reported to be ~120 µl. SB 203580 (10 nmol) increased the number of surviving RGCs from 348 to 1,347 cells/mm² 14 days post axotomy (control: 2,511 cells/mm²).

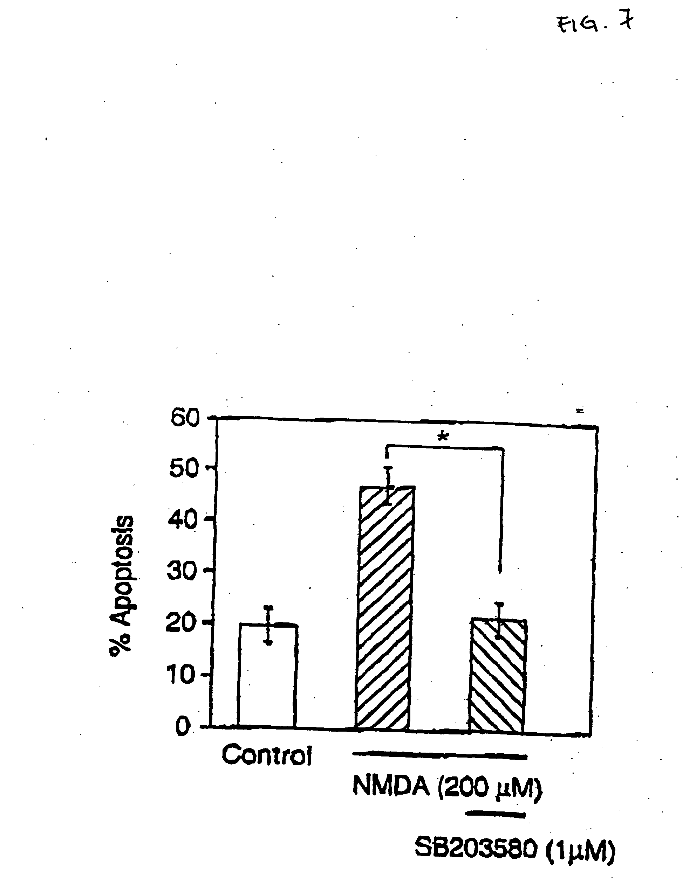

To determine whether glutamate neurotoxicity via activation of the NMDA receptor could effect apoptosis via the p38 signaling pathway in RGCs, the effects of SB203580 on NMDA-induced RGC apoptosis *in vitro* was examined. Referring to Fig. 7, treatment with 1 µM SB203580 significantly increased the survival of RGCs in the face of an NMDA exposure capable of causing apoptosis. This finding suggested that p38 activation is involved in RGC apoptosis mediated by the NMDA receptor.

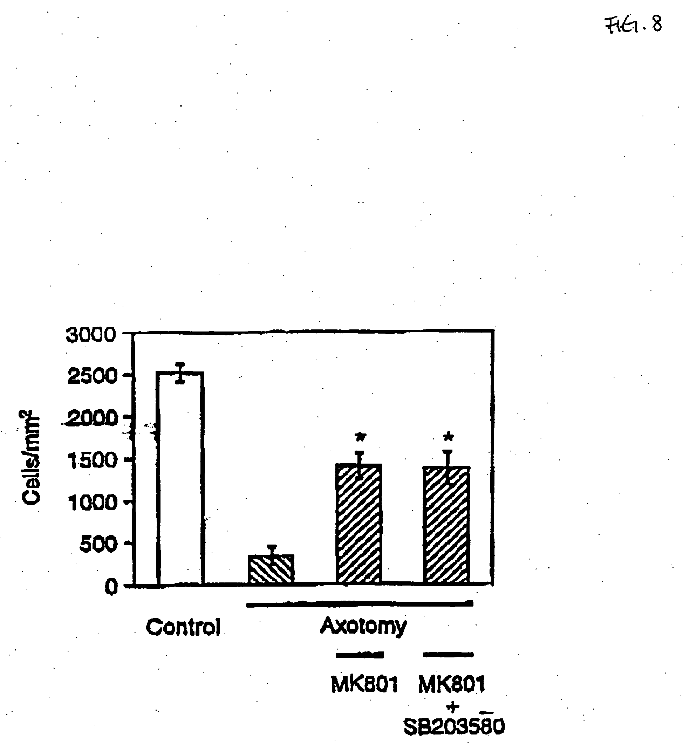

The effect on axotomy of the noncompetitive NMDA receptor antagonist MK801 *in vivo* was also investigated. MK801 was injected into the vitreous in the same manner as SB 203580. Referring to Fig. 8, MK801 not only increased the number of surviving RGCs 14 days post axotomy, but also inhibited p38 phosphorylation/activation in a dose-dependent manner. The addition of SB 203580 to MK801 did not offer further protection above that of MK801 alone in this paradigm.

Detailed procedures for carrying out the above-described experiments are set forth below.
***Retrograde labelireg of retinal ganglion cells.*** Aduh male Long-Evans rats weighing 200 to 250 g were obtained from a local breeder and, for all experimental manipulations, were anesthetized with 1-2 % isoflurane and 70% N₂O. RGCs were retrogradely labeled with hydroxystilbamidine (Molecular Probes, Eugene, OR, also known as Fluoro-Gold™) to allow accurate counting of cell bodies as described in Vorwerk et al., Invest. Ophthalmol. Vis. Sci. 40:813-816. Rats were anesthetized and placed in a small stereotactic instrument. The skull was exposed and kept dry. The bregma was identified and marked, and a small window was drilled above the right hemisphere, leaving the dura intact. Using a stereotactic measuring device and a Hamilton injector, hydroxystilbamidine solution was injected into four (4) regions of the right superior colliculus using the following coordinates from the bregma (anterior-posterior; medio-lateral; depth, all listed in mm): (1) -5.8, +1.0, -4.4; (2) -6.5, +0.7, -4.0; (3) -6.5, +1.0, -4.0; and (4) -7.3, +1.5, -3.7.
***Axotomy.*** Optic nerve axotomy was performed on the left eye 4 days after retrograde labeling. After incision of the dorsolateral conjunctiva, the lateral extraocular muscle was transected, and the optic nerve was exposed under a stereoscopic microscope. The optic nerve was then transected at a distance of about 1 mm from the eye bulb. During the operation, care was taken to avoid damage to the retinal blood supply.
***Drug application.*** Intravitreal injections were carried out using a 33-gauge needle attached to a 25 µl syringe following pupil dilation with 1% atropine sulfate. The tip of the needle was inserted through the dorsal limbus of the eye under stereomicroscopic visualization. Injections were completed over a period of 1 min. Intravitreal injections of SB 203580 (Calbiochem, San Diego, CA), MK801 (Research Biochemicals International, Natick, MA) or control solutions (as an equal volume of saline diluent) were performed on operated eyes immediately after axotomy, and repeated 5 and 10 days post axotomy.
***Quantification of axotomized RGC survival and histology.*** At various time points, rats were given an overdose of pentobarbital, and the eyes were removed. The retina was carefully dissected from the eye, prepared as a flat whole mount in a 4% paraformaldehyde solution, and examined for stained ganglion cells by epifluorescence microscopy to determine the density. The number of surviving RGCs in experimental and control retinas was determined by counting hydroxystilbamidine-labeled neurons in three standard areas of each retinal quadrant at one-sixth, one-half and five-sixths of the retinal radius, for a total area of 2.25 mm² as described in Kermer et al., J Neurosci 18:4656-4662 (1998). RGC survival data from each group of animals is presented as the mean density (RGCs/mm²) and standard deviation for n = 3 to 6 retinas. Statistical significance of the data was determined by an analysis of variance (ANOVA) followed by a post-hoc Dunnett's test. For the histological studies, retinal whole mounts were prepared and stained by the method ofNissl using cresyl violet (0.1%).
***Immunohistochemistry.*** After enucleation, the eyes were immersed in fixative composed of 4% paraformaldehyde in phosphate-buffered saline (PBS; pH 7.4) at 4 °C. Ten minutes later, the eyes were hemisected in the fixative, and the anterior segment, lens and vitreous body were discarded. The remaining posterior eyecup was kept in fresh fixative solution overnight at 4°C. The eyecup was then embedded in paraffin. Sections 5 µm in thickness were cut on a microtome and transferred onto gelatin-coated glass slides. After rehydration, sections were treated for 30 min at room temperature with methanol to increase membrane permeability, followed by 4% hydrogen peroxide for 1 hr to block intrinsic peroxidase activity. Then, sections were incubated with 20% normal goat serum (NGS) for 1 hr. After rinsing, the sections were incubated overnight at 4°C in PBS with 0.3 % Triton-X 100, 1 % NGS, and one of the following specific antibodies: 1:1000 anti-p38α antibody (Santa Cruz Biotechnology, Inc., Santa Cruz, CA), 1:250 anti-phospho-specific p38 (New England BioLabs Inc., Beverly, MA), or 1:50 anti-rat ED1 antibody (Serotech, UK). The sections were then incubated with biotinylated anti-IgG (Sigma Immunochemicals) for 2 hr at room temperature. Color development was performed with a Vector AEC substrate kit (Vector Laboratories Inc., Burlingame, CA) or with a Sigma Fast DAB kit. When immunoreactivity was exclusively localized to the nucleus, counterstaining was needed to define the cell somata, and Meyer Hematoxylin was used.
***Irnmunoblotting.*** Retinas were homogenized in sodium dodecyl sulfate (SDS) sample buffer (2% SDS, 0.6% 2-mercaptoethanol, 10% glycerol, 50 mM Tris, pH 7.2) and centrifuged. After estimation of supernatant protein concentration with a BIO-RAD Protein Assay™ (Bio-Rad Lab., Hercules, CA), aliquots containing 70 µg of protein were separated by SDS-PAGE and transferred onto a nitrocellulose membrane (Hybond ECL, Amercham Pharmacia Biotech, UK). The membranes were then blocked with 25 mM Tris-HCl (pH 7.4), 137 mM NaCl, 2.68 mM KCl and 0.1 % Tween 20 containing 5% nonfat milk for 1 hr at room temperature.
Membranes were probed with 1:1500 anti-p38a antibody, 1:200 anti-p38β antibody (Santa Cruz Biotechnology), or 1:500 anti-phospho-specific p38 according to the instructions of the manufacturer. The antibody-reactive bands were visualized by chemiluminescent detection (ECL western detection kit, Amercham Pharmacia Biotech UK, Limited).
***Retinal cell cultures.*** Retinal cells were prepared from 6-10 day old Long-Evans rats as described in Leifer et al., Science 224:303-306 (1984). Briefly, following dissociation in papain, retinal cells were plated on poly-L-lysine-coated-glass coverslips in Eagle's minimum essential medium. RGCs were identified by immunocytochemical staining using an anti-Thy-1 antibody (2G12), which is specific among rat retinal cells for the ganglion cells.
***Assessment of NWDA-induced apoptosis in cultured RGCs.* To induce** predominantly apoptosis (See Bonfoco et al., Proc. Natl. Acad. Sci. USA 92:7162-7166 (1995); Dreyer et al., Neuroreport 6:942-944 (1995)), retinal cell cultures were exposed to 300 µM NMDA/5 µM glycine for 18 hr in high calcium (3 mM) medium. For specific labeling of RGCs, the cultures were incubated with anti-Thy-1 antibody for 1 hr. Following 3 washes with PBS, cells were incubated in goat-anti-mouse-IgG-FITC for 1 hr. For assessment of apoptosis, retinal cells were fixed, permeabilized and stained with 20 µg/ml propidium iodide for 5 min, as described in Ankarcrona et al., Neuron 15:961-973 (1995). Briefly, coverslips containing the cells were washed once with PBS and permeabilized with 85% methanol for 10 min. After another wash with PBS, coverslips were fixed in acetone for 5 min and subsequently stained with propidium iodide for 5 min in the dark. The coverslips were then mounted on glass slides in glycerol:PBS (1:1), and visualized under epifluorescence microscopy. Apoptotic nuclei were scored in cells that were also stained by anti- Thy-1, and expressed as a fraction of total RGCs.

The p38 MAPK inhibitor may be included in a pharmaceutical preparation, using a pharmaceutical carrier (e.g., physiological saline); the exact formulation of the therapeutic mixture depends upon the route of administration, e.g., orally, parenterally (intravenous, intramuscular, intraperitoneal, subcutaneous), intravitreally, topically instilled into the eye, intracerebroventricularly, or intrathecally.

Examples of parenteral dosage forms include aqueous solutions of the active agent, in a isotonic saline, 5% glucose or other well-known pharmaceutically acceptable excipient. Solubilizing agents such as cyclodextrins, or other solubilizing agents well-known to those familiar with the art, can be utilized as pharmaceutical excipients for delivery of the therapeutic compounds. For oral administration, the p38 MAPK inhibitor composition can be formulated in a capsule, a gel seal or a tablet. Capsules may comprise any standard pharmaceutically acceptable material such as gelatin or a cellulose derivative. Tablets may be formulated in accordance with the conventional procedure by compressing mixtures of the p38 MAPK inhibitors and a solid carrier, and a lubricant. Examples of solid carriers include starch and sugar bentonite. The p38 MAPK inhibitor can also be administered in a form of a hard shell tablet or capsule containing, for example, lactose or mannitol as a binder and a conventional filler and a tableting agent. For compositions to be delivered to the eyes, topical formulations can be used and can include ophthalmoligically acceptable preservatives, surfactants viscosity enhancers, buffers, sodium chloride, and water to form a sterile ophthalmic solutions and suspension. An effective amount of a p38 MAPK inhibitor is defined as the amount of the compound which, upon administration to a patient in need, confers a therapeutic effect on the treated patient. The effective amount to be administered to a patient is typically based on age, surface area, weight, and condition of the patient. The interrelationship of dosages for animals and humans (based on milligrams per meter squared of body surface) is described by Freireich et al., Cancer Chemother. Rep. 1966, 50, 219. Body surface area may be approximately determined from height and weight of the patient. See, e.g., Scientific Tables, Geigy Pharmaceuticals, Ardley, New York, 1970, 537. An effective amount of a p38 MAPK inhibitor can range from about 1-10,000 mg/kg (e.g., about 1-500 mg/kg). Effective doses will also vary, as recognized by those skilled in the art, dependant on route of administration, excipient usage, and the possibility of co-usage with other therapeutic treatments.

## Claims

1. Use of a p38 mitogen-activated protein kinase (MAPK) inhibitor in the manufacture of a medicament for treating a patient suffering from glaucoma, optic neuropathy, optic neuritis, retinal ischemia, laser induced optic damage, or surgery-or trauma-induced proliferative vitreoretinopathy; a patient suffering neuronal injury or apoptosis involving retinal ganglion cells; or a patient suffering from a medical condition which causes nitrosative or oxidative stress to retinal ganglion cells by excessive activation of excitatory amino acid receptors or the generation of free radicals.

2. Use according to Claim 1, wherein the p38 MAPK inhibitor is of the following formula: 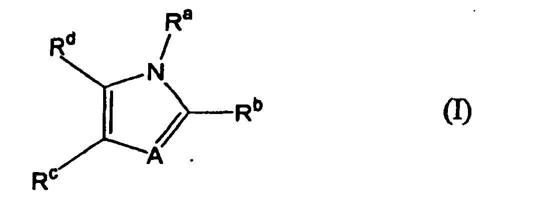 wherein
A is N or CR^{α}, and
each of R^{α}, R^{a}, R^{b}, and R^{c}, independently, is hydrogen, alkyl, hydroxyl, alkoxy, aryloxy, heteroaryloxy, thio, amino, amide, carboxyl, ester, amide, halo, cycloalkyl, heterocycloalkyl, aryl, heteroaryl, aralkyl, or heteroaralkyl, each of cycloalkyl, heterocycloalkyl, aryl, and heteroaryl being optionally substituted with alkyl, hydroxyl, alkoxy, aryloxy, heteroaryloxy, thio, amino, amide, carboxyl, ester, alkylsulfinyl, or halo;
R^{c} and R^{d} optionally joining together to form cycloalkyl, heterocycloalkyl, aryl, or heteroaryl, each of which being optionally substituted with alkyl, hydroxyl, alkoxy, aryloxy, heteroaryloxy, thio, amino, amide, carboxyl, ester, alkylsulfinyl, or halo;
or a salt thereof.

3. Use according to Claim 2, wherein A is N, and R^{a} is hydrogen, cycloalkyl, heterocycloalkyl, aryl, heteroaryl, aralkyl, or heteroaralkyl.

4. Use according to Claim 2, wherein A is N, and R^{b} is hydrogen, aryl, or heteroaryl.

5. Use according to Claim 4, wherein R^{b} is phenyl, substituted at the 4-position with alkoxy, alkylsulfinyl, halo, or amino.

6. Use according to Claim 2, wherein A is N, and R^{c} is hydrogen, aryl, or heteroaryl.

7. Use according to Claim 6, wherein R^{c} is phenyl, substituted with alkoxy, alkylsulfinyl, halo, or amino.

8. Use according to Claim 2, wherein A is N and R^{d} is 4-pyridyl, preferably unsubstituted.

9. Use according to Claim 2, wherein A is CR^{α}, and R^{c} and R^{d} join together to form heteroaryl which is optionally substituted with alkyl, alkoxy, aryloxy, amino, or halo.

10. Use according to Claim 1, wherein the P38 MAPK inhibitor is 4-(4-fluorophenyl)-2-(4-methylsulfinylphenyl)-5-(4-pyridyl)-*1H*-imidazole, 4-(4-fluorophenyl)-2-(4-hydroxylphenyl)-5-(4-pyridyl)-*1H*-imidazole, 5-(2-amino-4-pyrimidinyl)-4-(4-fluorophenyl)-1-(4-piperidinyl)-*1H*-imidazole, 2-methyl-4-phenyl-5-(4-pyridyl)oxazole, SmithKline French drug no. SKF-104,351, 6-(4-fluorophenyl)-2,3-dihydro-5-(4-pyridyl)-imidazo-[2,1-b]-thiazole, 6-amino-2-(4-fluorophenyl)-4-methoxy-3-(4-pyhdyl)-*1H*-pyrrolo[2,3-b]pyhdine, or 1-(4-piperidinyl)-4-(4-fluorophenyl)-5-(4-pyridyl)-*1H*-imidazole.

## Patentansprüche

1. Verwendung eines mitogen-aktivierten Proteinkinase-p38(MAPK)-Inhibitors bei der Herstellung eines Medikaments zur Behandlung eines Patienten, der an Glaukom, optischer Neuropathie, optischer Neuritis, retinaler Ischämie, Laser induziertem optischen Schaden oder Chirurgie oder Trauma induzierter proliferativer Vitreoretinopathie leidet; eines Patienten, der an neuronaler Verletzung oder Apoptose leidet, die retinale Ganglienzellen involviert; oder eines Patienten, der an einem medizinischen Zustand leidet, der nitrosativen oder oxidativen Stress an Ganglienzellen durch übermässige Aktivierung von anregenden Aminosäurerezeptoren oder der Generierung freier Radikale verursacht.

2. Verwendung gemäß Anspruch 1, wobei der p38-MAPK-Inhibitor der folgenden Formel ist: 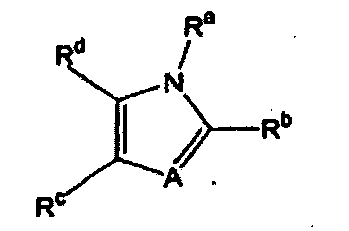 wobei
A N oder CR^{α} ist, und
jedes R^{α}, R^{a}, R^{b} und R^{c}, unabhängig, Hydrogen, Alkyl, Hydroxyl, Alkoxy, Aryloxy, Heteroaryloxy, Thio, Amino, Amid, Carboxyl, Ester, Amid, Halo, Cycloalkyl, Heterocycloalkyl, Aryl, Heteroaryl, Aralkyl oder Heteroaralkyl ist, jedes Cycloalkyl, Heterocycloalkyl, Aryl und Heteroaryl optional durch Alkyl, Hydroxyl, Alkoxy, Aryloxy, Heteroaryloxy, Thio, Amino, Amid, Carboxyl, Ester, Alkylsulfinyl oder Halo substituiert wird;
R^{c} und R^{d} sich optional verbinden, um Cycloalkyl, Heterocycloalkyl, Aryl, oder Heteroaryl zu bilden, wovon jedes optional durch Alkyl, Hydroxyl, Alkoxy, Aryloxy, Heteroaryloxy, Thio, Amino, Amid, Carboxyl, Ester, Alkylsulfinyl oder Halo;
oder einem Salz davon substituiert wird.

3. Verwendung gemäß Anspruch 2, wobei A N ist und R^{a} Hydrogen, Cycloalkyl, Heterocycloalkyl, Aryl, Heteroaryl, Aralkyl oder Heteroaralkyl ist.

4. Verwendung gemäß Anspruch 2, wobei A N ist und R^{b} Hydrogen, Aryl oder Heteroaryl ist.

5. Verwendung gemäß Anspruch 4, wobei R^{b} Phenyl , an der 4-Position durch Alkoxy, Alkylsulfinyl, Halo oder Amino substituiert, ist.

6. Verwendung gemäß Anspruch 2, wobei A N ist und R^{c} Hydrogen, Aryl oder Heteroaryl ist.

7. Verwendung gemäß Anspruch 6, wobei R^{c} Phenyl, substituiert durch Alkoxy, Alkylsulfinyl, Halo oder Amino, ist.

8. Verwendung gemäß Anspruch 2, wobei A N ist und R^{d} 4-Pyridyl, vorzugsweise nichtsubstituiert, ist.

9. Verwendung gemäß Anspruch 2, wobei A CR^{α} ist und sich R^{c} und R^{d} verbinden, um Heteroaryl zu bilden, das optional durch Alkyl, Alkoxy, Aryloxy, Amino oder Halo substiotuiert wird.

10. Verwendung gemäß Anspruch 1, wobei der P38-MAPK-Inhibitor 4-(4-Fluorophenyl)-2-(4-Methylsulfmylphenyl)-S-(4-Pyridyl)-IH-Imidazol, 4-(4-Fluorophenyl)-2-(4-Hydroxylphenyl)-5-(4-Pyridyl)-IH-Imidazol, 5-(2-Amino-4-Pyrimidinyl)-4-(4-Fluorophenyl)-1-(4-Piperidinyl)-IH-Imidazol, 2-Methyl-4-Phenyl-5-(4-Pyridyl)Oxazol, SmithKline French Droge Nr. SKF-104,351, 6-(4-FIuorophenyl)-2,3-Dihydro-5-(4-Pyndyl)-tmidazo-[2,1-b]-Thiazole, 6-Amino-2-(4-Fluorophenyl)-4-Methoxy-3-(4-Pyridyl)-IH-Pyrrolo[2,3-b]Pyridin, oder 1-(4 - Piperidinyl)-4-(4-Fluorophenyl)-5-(4-Pyridyl)-1 H-Imidazol ist.

## Revendications

1. Utilisation d'un inhibiteur d'une protéine kinase activée par le mitogène p38 (MAPK) dans la fabrication d'un médicament conçu pour traiter un patient souffrant d'un glaucome, d'une neuropathie optique, d'une névrite optique, d'une ischémie rétinienne, d'une lésion oculaire induite par un laser ou d'une rétinopathie vitreuse d'origine chirurgicale ou traumatique, d'un patient souffrant d'une lésion neuronale ou d'une apoptose mettant en jeu les cellules ganglionnaires rétiniennes ou d'un patient présentant un état pathologique médical qui cause un stress nitrosatif ou oxydatif des cellules ganglionnaires rétiniennes par l'activation excessive de récepteurs aux acides aminés excitateurs ou par la génération de radicaux libres.

2. Utilisation selon la revendication 1, où l'inhibiteur de la MAPK p38 répond à la formule : 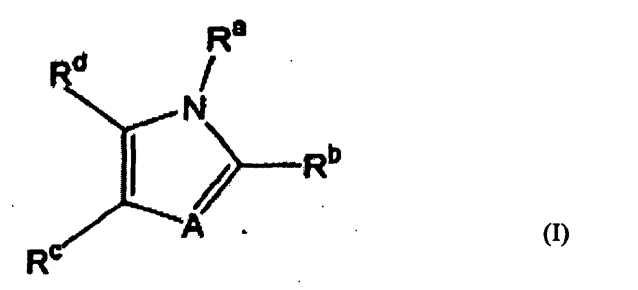 où
A représente N ou CR^{α} et
chaque R^{α}, R^{a}, R^{b} et R^{c} représente indépendamment un hydrogène ou un groupement alkyle, hydroxyle, alkoxy, aryloxy, hétéroaryloxy, thio, amino, amide, carboxyle, ester, amide, halo, cycloalkyle, hétérocycloalkyle, aryle, hétéroaryle, aralkyle ou hétéroaralkyle, chacun des groupements cycloalkyle, hétérocycloalkyle, aryle et hétéroaryle étant en option substitué par un groupement alkyle, hydroxyle, alkoxy, aryloxy, hétéroaryloxy, thio, amino, amide, carboxyle, ester, alkylsulfinyle ou halo ;
R^{c} et R^{d} sont en option joints pour former un groupement cycloalkyle, hétérocycloalkyle, aryle ou hétéroaryle chacun en option substitué par un groupement alkyle, hydroxyle, alkoxy, aryloxy, hétéroaryloxy, thio, amino, amide, carboxyle, ester, alkylsulfinyle ou halo ;
ou un sel dudit composé.

3. Utilisation selon la revendication 2, où A représente N et R^{a} un hydrogène ou un groupement cycloalkyle, hétérocycloalkyle, aryle, hétéroaryle, aralkyle ou hétéroaralkyle.

4. Utilisation selon la revendication 2, où A représente N et R^{b} un hydrogène ou un groupement aryle ou hétéroaryle.

5. Utilisation selon la revendication 4, où R^{b} représente un groupement phényle substitué en position 4 par un groupement alkoxy, alkylsulfinyle, halo ou amino.

6. Utilisation selon la revendication 2, où A représente N et R^{c} un hydrogène ou un groupement aryle ou hétéroaryle.

7. Utilisation selon la revendication 6, où R^{c} représente un groupement phényle substitué par un groupement alkoxy, alkylsulfinyle, halo ou amino.

8. Utilisation selon la revendication 2, où A représente N et R^{d} un groupement 4-pyridyle, de préférence non substitué.

9. Utilisation selon la revendication 2, où A représente CR^{α} et où R^{c} et R^{d} sont joints pour former un groupement hétéroaryle qui est en option substitué par un groupement alkyle, alkoxy, aryloxy, amino ou halo.

10. Utilisation selon la revendication 1, où l'inhibiteur de la MAPK p38 est le 4-(4-fluorophényl)-2-(4-méthylsulfinylphényl)-5-(4-pyridyl)-*1H*-imidazole, le 4-(4-fluorophényl)-2-(4-hydroxylphényl)-5-(4-pyridyl)-*1H*-imidazole, le 5-(2-amino-4-pyrimidinyl)-4-(4-fluorophényl)-1-(4-pipéridinyl)-*1H*-imidazole, le 2-méthyl-4-phényl-5-(4-pyridyl)oxazole, le composé N° SKF-104,351 de SmithKline French, le 6-(4-fluorophényl)-2,3-dihydro-5-(4-pyridyl)-imidazo-[2,1-b]-thiazole, la 6-amino-2-(4-fluorophényl)-4-méthoxy-3-(4-pyridyl)-*1H*-pyrrolo[2,3-b]pyridine ou le 1-(4-pipéridinyl)-4-(4-fluorophényl)-5-(4-pyridyl)-*1H*-imidazole.
